(19) Europäisches Patentamt
European Patent Office
Office européen des brevets

(11) Veröffentlichungsnummer : **0 399 285 B1**

(12) **EUROPÄISCHE PATENTSCHRIFT**

(45) Veröffentlichungstag der Patentschrift : **30.09.92 Patentblatt 92/40**

(51) Int. Cl.$^5$ : **C07D 249/14,** // A01N43/653

(21) Anmeldenummer : **90108629.8**

(22) Anmeldetag : **08.05.90**

(54) **Verfahren zur Herstellung von 3-Amino-5-aminocarbonyl-1,2,4-triazol-Derivaten.**

(30) Priorität : **20.05.89 DE 3916430**

(43) Veröffentlichungstag der Anmeldung : **28.11.90 Patentblatt 90/48**

(45) Bekanntmachung des Hinweises auf die Patenterteilung : **30.09.92 Patentblatt 92/40**

(84) Benannte Vertragsstaaten : **BE CH DE FR GB IT LI NL**

(56) Entgegenhaltungen : **EP-A- 0 126 326**

(56) Entgegenhaltungen : **CHEMICAL ABSTRACTS, Band 63, Nr. 10, 8. November 1965, Zusammenfassung 13243f - 13244b, Columbus, Ohio, US; G.E. CIPENS etal.: "Derivatives of 1,2,4-triazole-5-carboxylic acid", & LATVIJAS PSR ZINATNU AKAD. VESTIS, KIM. SER. 1965(2), 204-8**
**BULLETIN DE LA SOCIETE CHIMIQUE DE FRANCE, Nr. 4, April 1970, Seiten 1590-1599, Paris, FR; M. PESSON et al.: "Recherches surles dérivés du triazole-1,2,4. V. - Amides N-dialkylés d'acides triazol-1,2,4 yl-5 carboxyliques"**

(73) Patentinhaber : **BAYER AG**
**W-5090 Leverkusen 1 Bayerwerk (DE)**

(72) Erfinder : **Findeisen, Kurt, Dr.**
**Dünfelder Strasse 28**
**W-5090 Leverkusen 1 (DE)**
Erfinder : **Lindig, Markus, Dr.**
**Hildegardstrasse 9**
**W-4018 Langenfeld (DE)**

EP 0 399 285 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 3-Amino-5-aminocarbonyl-1,2,4-triazol-Derivaten, welche als neue, herbizid wirksame Stoffe Gegenstand einer älteren, nicht vorveröffentlichten Patentanmeldung sind (vgl. DE-P 3809053 vom 18. März 1988).

Der genannten Patentanmeldung ist zu entnehmen, daß man 3-Amino-5-aminocarbonyl-1,2,4-triazol-Derivate erhält, wenn man (a) geeignete Aminoguanidine mit Oxalesteramiden oder (b) geeignete Triazolylcarbonsäureester mit Aminen umsetzt. Nach beiden Methoden erhält man die 3-Amino-5-aminocarbonyl-1,2,4-triazol-Derivate in vielen Fällen nur in unbefriedigenden Ausbeuten.

Es wurde nun gefunden, daß man 3-Amino-5-aminocarbonyl-1,2,4-triazol-Derivate der allgemeinen Formel (I)

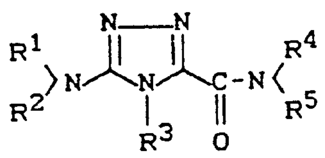

(I)

in welcher

$R^1$ für Wasserstoff, Methyl oder Ethyl steht,

$R^2$ für Methyl oder Ethyl steht,

$R^3$ für Methyl oder Ethyl steht,

$R^4$ für Wasserstoff oder Methyl steht,

$R^5$ für Wasserstoff, Methyl, Ethyl, n- oder i-Propyl, n-, i-, s- oder t-Butyl, n- oder i-Pentyl, n - oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor und/oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden durch Fluor, Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht;

$R^5$ außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen jeweils infrage kommen:

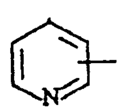 ; 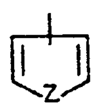 ; 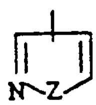 ; 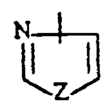 ;

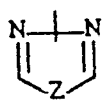 ; 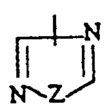 ; 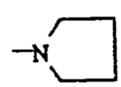 ; 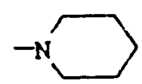 ;

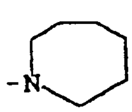 ; 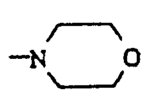 oder 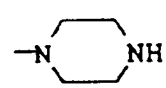 ,

wobei Z jeweils für Sauerstoff oder Schwefel steht,

$R^5$ außerdem für jeweils gegebenenfalls im Phenylteil ein- bis dreifach, gleich oder verschieden durch fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht.

in guten Ausbeuten und in hoher Reinheit erhält, wenn man Chlor-formamidin-Hydrochloride der allgemeinen Formel (II)

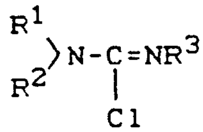 x HCl (II)

in welcher

$R^1$, $R^2$ und $R^3$ die oben angegebenen Bedeutungen haben,

mit Oxalsäure-amid-hydraziden der allgemeinen Formel (III)

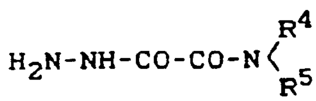 (III)

in welcher

$R^4$ und $R^5$ die oben angegebenen Bedeutungen haben,

in Gegenwart eines Säureakzeptors und in Gegenwart eines Verdünnungsmittels bei Temperaturen zwischen 0°C und 150°C umsetzt.

Die nach dem erfindungsgemäßen Verfahren herzustellenden substituierten Triazole sind durch die Formel (I) allgemein definiert. Bevorzugt sind Verbindungen der Formel (I), bei welchen

$R^1$ für Wasserstoff, Methyl oder Ethyl steht,

$R^2$ für Methyl oder Ethyl steht,

$R^3$ für Methyl oder Ethyl steht,

$R^4$ für Wasserstoff oder Methyl steht,

$R^5$ Wasserstoff, Methyl, Ethyl, n- oder i- Propyl, n-, i-, s- oder t-Butyl, n- oder i-Pentyl, n- oder i- Hexyl, Allyl, n- oder i-Butenyl, n- oder i-Pentenyl, Propargyl, n- oder i-Butinyl, n- oder i-Pentinyl, Halogen-$C_1$-$C_6$-alkyl mit 1 bis 6 gleichen oder verschiedenen Halogenatomen, insbesondere Fluor und Chlor, Halogen-$C_3$-$C_5$-alkenyl oder Halogen-$C_3$-$C_5$-alkinyl mit jeweils 1 bis 3 Halogenatomen, insbesondere Fluor und/oder Chlor, jeweils gegebenenfalls einfach bis dreifach gleich oder verschieden substituiertes Cyclopropyl, Cyclopentyl, Cyclohexyl, Cyclopropylmethyl, Cyclopentylmethyl oder Cyclohexylmethyl bedeutet, wobei als Substituenten Fluor, Chlor, Brom, Methyl und Ethyl ausgewählt sind.

Im einzelnen seien außer den bei den Herstellungsbeispielen genannten Verbindungen die folgenden Verbindungen der allgemeinen Formel (I) genannt:

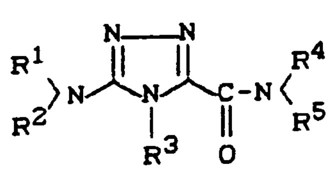 (I)

| $R^1$ | $R^2$ | $R^3$ | $R^4$ | $R^5$ |
|---|---|---|---|---|
| $CH_3$ | $CH_3$ | $C_2H_5$ | H | $-C(CH_3)_3$ |
| H | $CH_3$ | $CH_3$ | H | 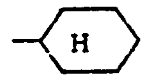 |
| H | $CH_3$ | $C_2H_5$ | H | $-CH_2-C(CH_3)_3$ |
| H | $CH_3$ | $C_2H_5$ | H | 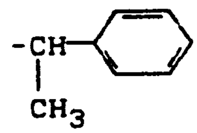 |
| $CH_3$ | $CH_3$ | $CH_3$ | $CH_3$ | $-C(CH_3)_3$ |
| $CH_3$ | $CH_3$ | $C_2H_5$ | $CH_3$ | $-C(CH_3)_3$ |
| $C_2H_5$ | $C_2H_5$ | $CH_3$ | H | 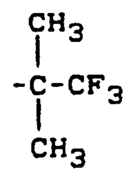 |
| H | $C_2H_5$ | $CH_3$ | H | $-C(CH_3)_3$ |
| H | $CH_3$ | $CH_3$ | $CH_3$ | $-C(CH_3)_3$ |
| $CH_3$ | $CH_3$ | $CH_3$ | H | 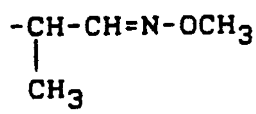 |
| $CH_3$ | $CH_3$ | $CH_3$ | $CH_3$ | 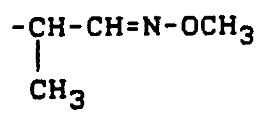 |
| H | $CH_3$ | $CH_3$ | H | 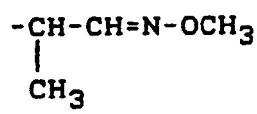 |

Verwendet man beispielsweise Chlortrimethylformamidin-Hydrochlorid und Oxalsäure-hydrazidmethylamid als Ausgangsstoffe, so kann der Reaktionsablauf beim erfindungsgemäßen Verfahren durch das folgende Formelschema wiedergegeben werden:

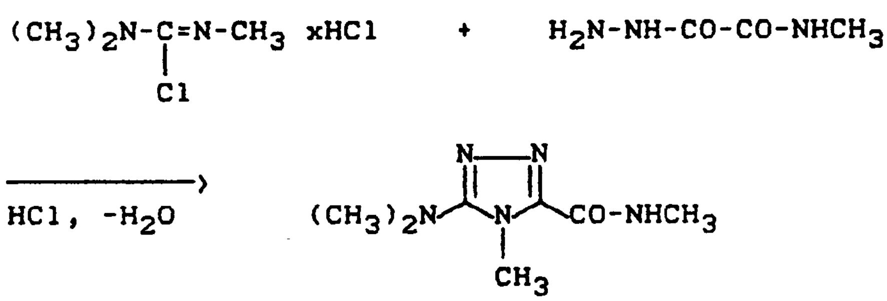

Die beim erfindungsgemäßen Verfahren zur Herstellung von Verbindungen der Formel (I) als Ausgangsstoffe zu verwendenden Chlor-formamidin-Hydrochloride sind durch die Formel (II) allgemein definiert.

In Formel (II) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Als Beispiele für die Ausgangsstoffe der Formel (II) seien genannt:

Chlor-N,N′-dimethyl-formamidin-Hydrochlorid, Chlor-N,N,N′-trimethyl-formamidin-Hydrochlorid, Chlor-N,N,N′-triethyl-formamidin-Hydrochlorid, Chlor-N,N,N′-tripropyl-formamidin-Hydrochlorid, Chlor-N,N,N′-triisopropyl-formamidin-Hydrochlorid, Chlor-N,N,N′-tributyl-formamidin-Hydrochlorid, Chlor-N,N-dimethyl-N′-ethyl-formamidin-Hydrochlorid, Chlor-N,N-dimethyl-N′-propyl-formamidin-Hydrochlorid, Chlor-N,N-dimethyl-N′-isopropyl-formamidin-Hydrochlorid, Chlor-N,N-dimethyl-N′-butyl-formamidin-Hydrochlorid, Chlor-N,N-dimethyl-N′-isobutyl-formamidin-Hydrochlorid, Chlor-N,N-dimethyl-N′-sec-butyl-formamidin-Hydrochlorid, Chlor-N,N-dimethyl-N′-tert-_ formamidin-Hydrochlorid, Chlor-N,N-diethyl-N′-methyl-formamidin-Hydrochlorid, Chlor-N,N-diethyl-N′-propyl-formamidin-Hydrochlorid, Chlor-N,N-diethyl-N′-butyl-formamidin-Hydrochlorid, Chlor-N,N-dipropyl-N′-methyl-formamidin-Hydrochlorid und Chlor-N,N-dipropyl-N′-ethyl-formamidin-Hydrochlorid.

Die Ausgangsstoffe der Formel (II) sind bekannt und/oder können nach an sich bekannten Verfahren hergestellt werden (vgl. DE-OS 3709574; Chem. Ber. 97 (1964), 1232-1245).

Die beim erfindungsgemäßen Verfahren weiter als Ausgangsstoffe zu verwendenden Oxalsäure-amid-hydrazide sind durch die Formel (III) allgemein definiert.

In Formel (III) haben $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$ und $R^5$ angegeben wurden.

Als Beispiele für die Ausgangsstoffe der Formel (III) seien genannt:

Oxalsäure-hydrazid-methylamid, -ethylamid, -propylamid, -isopropylamid, -butylamid, -isobutylamid, -sec-butylamid, -tert-butylamid, -pentylamid, -isopentylamid, -sec-pentylamid, -tert-pentylamid, -hexylamid, -isohexylamid, -octylamid, -2-fluor-ethylamid, -2-chlor-ethylamid, -2,2,2-trifluor-ethylamid, -(1,1-bis-fluormethyl)-ethylamid, -1,1-dimethyl-2,2,2-trifluor-ethylamid, -1-cyano-2,2-dimethyl-propylamid, -2-cyano-ethylamid, -allylamid, -crotylamid, -propargylamid, -1-methyl-propargylamid, -1,1-dimethyl-propargylamid, -cyclopropylamid, -1-methyl-cyclopropylamid, -cyclobutylamid, -1-methyl-cyclobutylamid, -cyclopentylamid, -1-methyl-cyclopentylamid, -cyclohexylamid, -1-methyl-cyclohexylamid, -2-methyl-cyclohexylamid, -3-methyl-cyclohexylamid, -4-methyl-cyclohexylamid, -benzylamid, -4-methyl-benzylamid, -1-phenyl-ethylamid, -2-phenyl-ethylamid und -1-cyano-1-methyl-propylamid.

Die Ausgangsstoffe der Formel (III) sind bekannt und/oder können nach an sich bekannten Verfahren hergestellt werden (vgl. EP-A 126326).

Das erfindungsgemäße Verfahren wird in Gegenwart eines Verdünnungsmittels durchgeführt. Als Verdünnungsmittel werden vorzugsweise polare organische Lösungsmittel und/oder Wasser eingesetzt. Bevorzugte organische Lösungsmittel sind Alkohole wie Methanol, Ethanol, Propanol, Isopropanol und Butanol, Ether wie Ethylenglycol-dimethylether, Diethylenglycol-dimethylether, Tetrahydrofuran und Dioxan, Etheralkohole wie Ethylenglycol-monomethylether und -monoethylether, Amide wie Formamid und Dimethylformamid, Nitrile wie Acetonitril, Propionitril oder Benzonitril, sowie Pyridin. Methanol wird als Verdünnungsmittel besonders bevorzugt.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Chlor-formamidin-hydrochlorid der Formel (II) im allgemeinen zwischen 0,5 und 1,5 Mol, vorzugsweise zwischen 0,8 und 1,2 Mol, Oxalsäure-amid-hydrazid der Formel (III) und zwischen 1 und 5 Moläquivalenten, vorzugsweise zwischen 2 und 3 Moläquivalenten, eines Säureakzeptors ein.

Die Reaktionskomponenten der Formel (II) und (III) werden im allgemeinen bei Raumtemperatur mit dem

Verdünnungsmittel vermischt und nach Zugabe eines Säureakzeptors - gegebenenfalls bei erhöhter Temperatur - bis zum Ende der Umsetzung gerührt.

Die Aufarbeitung kann nach üblichen Methoden durchgeführt werden.

Im allgemeinen wird das Reaktionsgemisch nach Ende der Umsetzung eingeengt und der Rückstand mit Wasser und einem mit Wasser praktisch nicht mischbaren organischen Lösungsmittel, wie z.B. Methylenchlorid, ausgeschüttelt. Die organische Phase wird abgetrennt, mit einem Trockenmittel, wie z.B. Magnesiumsulfat, getrocknet und filtriert. Vom Filtrat wird das Lösungsmittel im Wasserstrahlvakuum sorgfältig abdestilliert. Der verbleibende Rückstand enthält im wesentlichen das Produkt der Formel (I), welches auf übliche Weise, beispielsweise durch Umkristallisation, weiter gereinigt werden kann.

Herstellungsbeispiele:

Beispiel 1

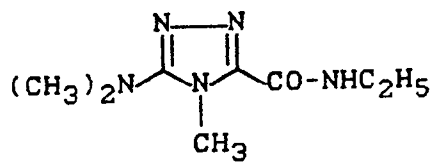

13,1 g (0,1 Mol, Oxalsäure-hydrazid-ethylamid werden bei 20°C zu einer Mischung aus 18,8 g (0,12 Mol, Chlortrimethylformamidin-hydrochlorid und 300 ml Methanol gegeben. Nach Zugabe von 30,3 g (0,3 Mol) Triethylamin steigt die Innentemperatur auf 44°C an. Das Reaktionsgemisch wird eine Stunde unter Rückfluß zum Sieden erhitzt und anschließend im Wasserstrahlvakuum eingeengt. Der Rückstand wird mit Methylenchlorid/Wasser ausgeschüttelt, die organische Phase abgetrennt, mit Natriumsulfat getrocknet und filtriert. Vom Filtrat wird das Lösungsmittel im Wasserstrahlvakuum sorgfältig abdestilliert.

Man erhält 12,8 g (65% der Theorie) 5-Dimethylamino-4-methyl-4H-1,2,4-triazol-3-yl-carbonsäureethylamid als kristallinen Rückstand vom Schmelzpunkt 73°C-75°C.

Beispiel 2

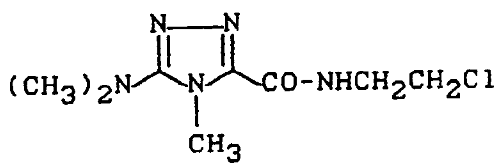

16,65 g (0,1 Mol) Oxalsäure-hydrazid-(2-chlor-ethyl)-amid werden bei 20°C zu einer Mischung aus 18,8 g (0,12 Mol) Chlortrimethylformamidin-hydrochlorid und 300 ml Methanol gegeben. Nach Zugabe von 16,2 g (0,3 Mol) Natriummethylat steigt die Innentemperatur auf 62°C an. Das Reaktionsgemisch wird eine Stunde ohne weiteres Erwärmen gerührt und anschließend im Wasserstrahlvakuum eingeengt. Der Rückstand wird mit Methylenchlorid/Wasser ausgeschüttelt, die organische Phase abgetrennt, mit Magnesiumsulfat getrocknet und filtriert. Vom Filtrat wird das Lösungsmittel im Wasserstrahlvakuum sorgfältig abdestilliert.

Man erhält 18,5 g (80% der Theorie) 5-Dimethylamino-4-methyl-4H-1,2,4-triazol-3-yl-carbonsäure-(2-chlor-ethyl)-amid als kristallinen Rückstand vom Schmelzpunkt 153°C-155°C.

Beispiel 3

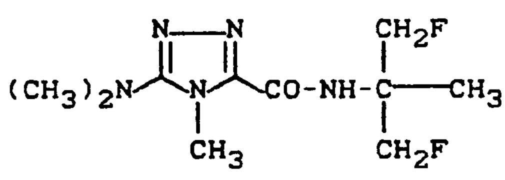

Analog zu Beispiel 2 erhält man aus 19,5 g (0,1 Mol) Oxalsäure-hydrazid-(1,1-bis-fluormethyl)-ethylamid

und 18,8 g (0,12 Mol) Chlortrimethylformamidin-hydrochlorid 19,7 g (75,5% der Theorie) 5-Dimethylamino-4-methyl-4H-1,2,4-triazol-3-yl-carbonsäure-(1,1-bis-fluormethyl)-ethylamid.

$^1$H-NMR ($CDCl_3$, δ, ppm): 1,55; 2,9; 4,5-4,8.

Beispiel 4

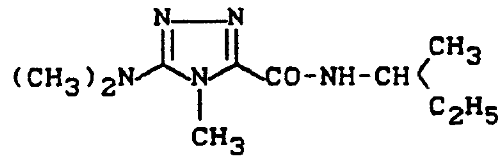

Analog zu Beispiel 2 erhält man aus 11,0 g (0,067 Mol) Oxalsäure-hydrazid-sec-butylamid und 10,8 g (0,069 Mol) Chlortrimethylformamidin-hydrochlorid 12,0 g (80% der Theorie) 5-Dimethylamino-4-methyl-4H-1,2,4-triazol-3-yl-carbonsäure-sec-butylamid vom Siedepunkt 149°C-152°C (bei 0,1 mbar).

$^1$H-NMR ($CDCl_3$, δ, ppm): 1,20-1,25; 2,85; 3,95-4,07.

Beispiel 5

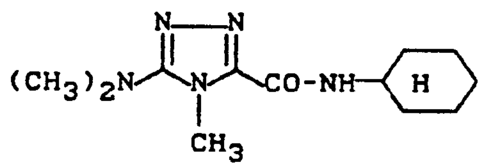

Analog zu Beispiel 2 erhält man aus 13,8 g (0,074 Mol) Oxalsäure-hydrazid-cyclohexylamid und 14,1 g (0,09 Mol) Chlortrimethylformamidin-hydrochlorid 16,0 g (86% der Theorie) 5-Dimethylamino-4-methyl-4H-1,2,4-triazol-3-yl-carbonsäure-cyclohexylamid vom Schmelzpunkt 60°C-62°C.

Beispiel 6

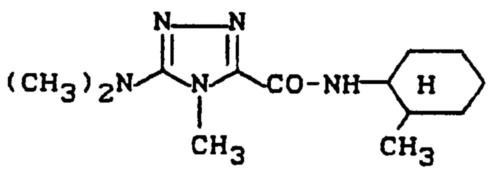

Analog zu Beispiel 2 erhält man aus 10,0 g (0,05 Mol) Oxalsäure-hydrazid-(2-methyl-cyclohexyl)-amid und 9,42 g (0,06 Mol) Chlortrimethylformamidin-hydrochlorid 11,2 g (84,5% der Theorie) 5-Dimethylamino-4-methyl-4H-1,2,4-triazol-3-yl-carbonsäure-(2-methyl-cyclohexyl)-amid.

$^1$H-NMR ($CDCl_3$, δ, ppm): 0,90-0,95; 2,85.

Beispiel 7

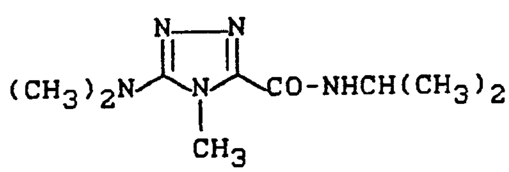

16,2 g (0,3 Mol) Natrium-methylat werden unter Rühren zu einer Mischung aus 18,8 g (0,12 Mol) Chlortrimethyl-formamidin-hydrochlorid, 14,5 g (0,10 Mol) Oxalsäure-hydrazid-isopropylamid und 200 ml Methanol gegeben und das Reaktionsgemisch wird 2 Stunden unter Rückfluß zum Sieden erhitzt. Nach dem Abkühlen wird abgesaugt, das Filtrat eingeengt und der Rückstand mit Methylenchlorid/Wasser ausgeschüttelt. Die organische Phase wird abgetrennt, mit Natriumsulfat getrocknet und filtriert. Das Filtrat wird durch Vakuumdestillation aufgearbeitet.

Man erhält 16,0 g (76% der Theorie) 5-Dimethylamino-4-methyl-4H-1,2,4-triazol-3-yl-carbonsäure-isopropylamid vom Siedepunkt 133-135°C (bei 0,1 mbar).
$^1$H-NMR ($CDCl_3$, δ, ppm): 1,25; 4,2; 7,3.

## Patentansprüche

1. Verfahren zur Herstellung von 3-Amino-5-aminocarbonyl -1,2,4-triazol-Derivaten der Formel (I)

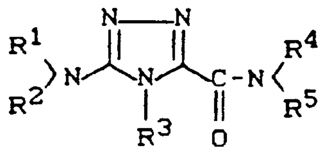

(I)

in welcher

$R^1$ für Wasserstoff, Methyl oder Ethyl steht,
$R^2$ für Methyl oder Ethyl steht,
$R^3$ für Methyl oder Ethyl steht,
$R^4$ für Wasserstoff oder Methyl steht,
$R^5$ für Wasserstoff, Methyl, Ethyl, n- oder i-Propyl, n-, i-, s- oder t-Butyl, n- oder i-Pentyl, n - oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor und/oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoximinoalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden durch Fluor, Chlor, Brom, Methyl, Ethyl, n- oder i-Propyl, n-, i-, s- oder t-Butyl, Cyano, Methandiyl, Ethandiyl, Butandiyl oder Butadiendiyl substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl, Cyclohexenylmethyl oder Cyclohexenylethyl steht;
$R^5$ außerdem für im Heterocyclylteil gegebenenfalls ein- bis dreifach, gleich oder verschieden durch Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio substituiertes Heterocyclylmethyl, Heterocyclylpropyl oder Heterocyclylethyl steht, wobei als Heterocyclen jeweils infrage kommen:

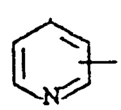 ; 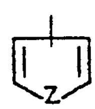 ; 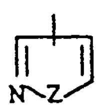 ; 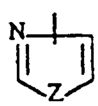 ;

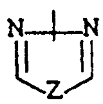 ; 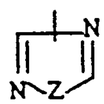 ; 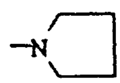 ; 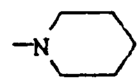 ;

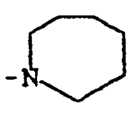 ; 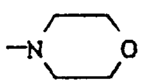 oder 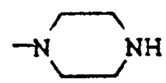 ,

wobei Z jeweils für Sauerstoff oder Schwefel steht,
$R^5$ außerdem für jeweils gegebenenfalls im Phenylteil ein- bis dreifach, gleich oder verschieden durch Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzoyl, Phenyl oder Naphthyl steht,
dadurch gekennzeichnet, daß man Chlor-formamidin-Hydrochloride der allgemeinen Formel (II)

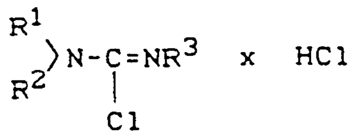 (II)

in welcher
$R^1$, $R^2$ und $R^3$ die oben angegebenen Bedeutungen haben,
mit Oxalsäure-amid-hydraziden der allgemeinen Formel (III)

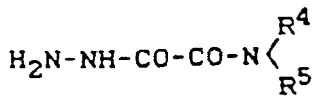 (III)

in welcher
$R^4$ und $R^5$ die oben angegebenen Bedeutungen haben,
in Gegenwart eines Säureakzeptors und in Gegenwart eines Verdünnungsmittels umsetz.

2. Verfahren gemäß Anpruch 1, dadurch qekennzeichnet, daß man die Umsetzunq bei Temperaturen von 0°C bis 150°C durchführt.

3. Verfahren gemäß Anspruch 1, dadurch gekennzeichnet, daß man auf 1 Mol Chlor-formamidin-hydrochlorid der Formel (II) zwischen 0,5 und 1,5 Mol Oxalsäure-amid-hydrazid der Formel (III) einsetzt.

4. Verfahren gemäß Anspruch 1, dadurch gekennzeichnet, daß man auf 1 Mol Chlor-formamidin-hydrochlorid der Formel (II) zwischen 0,5 und 1,5 Mol Oxalsäure-amidhydrazid der Formel (III) und 1 bis 5 Moläquivalente eines Säureakzeptors einsetzt.

## Claims

1. Process for the preparation of 3-amino-5-aminocarbonyl-1,2,4-triazole derivatives of the formula (I)

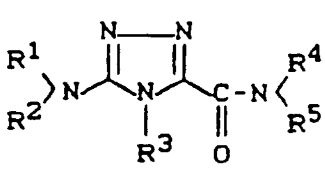 (I)

in which
$R^1$ represents hydrogen, methyl or ethyl,
$R^2$ represents methyl or ethyl,
$R^3$ represents methyl or ethyl,
$R^4$ represents hydrogen or methyl,
$R^5$ represents hydrogen, methyl, ethyl, n- or i- propyl, n-, i-, s- or t-butyl, n- or i-pentyl, n- or i-hexyl, n- or i-heptyl, n- or i-octyl, n- or i- nonyl, n- or i-decyl or n- or i-dodecyl, or represents allyl, n- or i-butenyl,

n- or i-pentenyl, n- or i-hexenyl, propargyl, n- or i-butinyl, n- or i- pentinyl or n- or i-hexinyl, or represents straight-chain or branched halogenoalkyl having 1 to 6 carbon atoms and 1 to 9 identical or different halogen atoms, in particular fluorine, chlorine or bromine, or represents in each case straight-chain or branched halogenoalkenyl or halogenoalkinyl, in each case having 3 to 5 carbon atoms and 1 to 3 halogen atoms, in particular fluorine and/or chlorine, or represents in each case straight-chain or branched cyanoalkyl having 1 to 6 carbon atoms in the alkyl moiety, hydroxyalkyl having 1 to 6 carbon atoms and 1 to 3 hydroxyl groups, or alkoxyalkyl, alkoximinoalkyl, alkoxycarbonylalkyl or alkoxycarbonylalkenyl, alkylaminoalkyl or dialkylaminoalkyl, in each case having up to 4 carbon atoms in the individual alkyl or alkenyl moieties, or represents cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl, cyclohexenylmethyl or cyclohexenylethyl, each of which is optionally monosubstituted to trisubstituted by identical or different substituents from the series comprising fluorine, chlorine, bromine, methyl, ethyl, n- or i-propyl, n-, i-, s- or t-butyl, cyano, methanediyl, ethanediyl, butanediyl or butadienediyl;

$R^5$ furthermore represents heterocyclylmethyl, heterocyclylpropyl or heterocyclylethyl, suitable heterocycles in each case being:

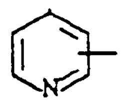 ; 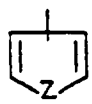 ; 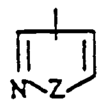 ; 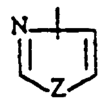 ;

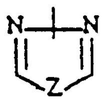 ; 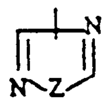 ; 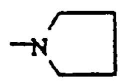 ; 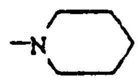 ;

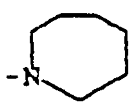 ; 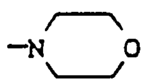 or 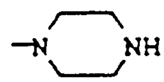 ,

where Z in each case represents oxygen or sulphur, each of which is optionally monosubstituted to trisubstituted in the heterocyclyl moiety by identical or different substituents from the series comprising fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^5$ furthermore represents benzyl, phenylethyl, phenylpropyl, phenylbutyl, phenylpentyl, phenylhexyl, phenylheptyl, phenylcyanomethyl, phenylcyanoethyl, phenylcyanopropyl, benzoyl, phenyl or naphthyl, if appropriate straight-chain or branched and in each case optionally monosubstituted to trisubstituted in the phenyl moiety by identical or different substituents from the series comprising fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy, characterized in that chloroformamidine hydrochlorides of the general formula (II)

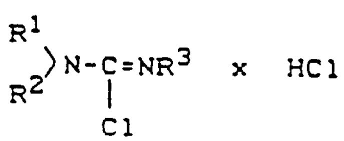 (II)

in which

$R^1$, $R^2$ and $R^3$ are as defined above,

are reacted with oxamohydrazides of the general formula (III)

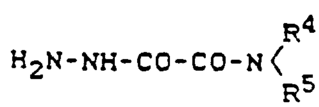 (III)

in which

$R^4$ and $R^5$ are as defined above,

in the presence of an acid acceptor and in the presence of a diluent.

2. Process according to Claim 1, characterized in that the reaction is carried out at temperatures from 0°C to 150°C.

3. Process according to Claim 1, characterized in that between 0.5 and 1.5 moles of oxamohydrazide of the formula (III) are employed per mole of chloroformamidine hydrochloride of the formula (II).

4. Process according to Claim 1, characterized in that between 0.5 and 1.5 moles of oxamohydrazide of the formula (III) and 1 to 5 mole equivalents of an acid acceptor are employed per mole of chloroformamidine hydrochloride of the formula (II).

## Revendications

1. Procédé de production de dérivés de 3-amino-5-aminocarbonyl-1,2,4-triazole de formule (I)

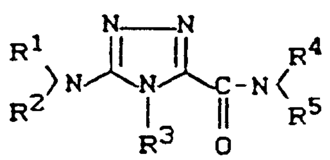

(I)

dans laquelle

$R^1$ représente l'hydrogène, un groupe méthyle ou éthyle,

$R^2$ est un groupe méthyle ou éthyle,

$R^3$ est un groupe méthyle ou éthyle,

$R^4$ est l'hydrogène ou un groupe méthyle

$R^5$ est l'hydrogène, un groupe méthyle, éthyle, n- ou isopropyle, n-, iso-, sec.- ou tertiobutyle, n- ou isopentyle, n- ou isohexyle, n- ou isoheptyle, n- ou iso-octyle, n- ou isononyle, n- ou isodécyle, n- ou isododécyle, un groupe allyle, n- ou isobutényle, n- ou isopentényle, n- ou isohexényle, propargyle, n- ou isobutynyle, n- ou isopentynyle, n- ou isohexynyle, un groupe halogénalkyle à chaîne droite ou à chaîne ramifiée ayant 1 à 6 atomes de carbone et 1 à 9 atomes d'halogènes identiques ou différents, en particulier fluor, chlore ou brome, un groupe halogénalcényle ou un groupe halogénalcynyle chacun à chaîne droite ou ramifiée et chacun avec 3 à 5 atomes de carbone et 1 à 3 atomes d'halogènes, notamment fluor et/ou chlore, un groupe cyanalkyle ayant 1 à 6 atomes de carbone dans la partie alkyle, hydroxyalkyle ayant 1 à 6 atomes de carbone et 1 à 3 groupes hydroxy, alkoxyalkyle, alkoxyminoalkyle, alkoxycarbonylalkyle ou alkoxycarbonylalcényle, chacun à chaîne droite ou ramifiée, un groupe alkylaminoalkyle ou un groupe dialkylaminoalkyle ayant chacun jusqu'à 4 atomes de carbone dans les parties alkyle ou alcényle individuelles ou un groupe cyclopropyle, cyclopropylméthyle, cyclopropyléthyle, cyclopentyle, cyclohexyle, cycloheptyle, cyclo-octyle, cyclohexylméthyle, cyclohexyléthyle, cyclohexényle, cyclohexénylméthyle ou cyclohexényléthyle portant chacun éventuellement 1 à 3 substituants fluoro, chloro, bromo, méthyle, éthyle, n- ou isopropyle, n-, iso-, sec.- ou tertiobutyle, cyano, méthanediyle, éthanediyle, butanediyle ou butadiènediyle, identiques ou différents ;

$R^5$ représente en outre un groupe hétérocyclylméthyle, hétérocyclylpropyle ou hétérocyclyléthyle portant éventuellement 1 à 3 substituants, identiques ou différents, fluoro, chloro, bromo, cyano, nitro, méthyle, éthyle, n- propyle ou isopropyle, n-, iso-, sec.- ou tertiobutyle, méthoxy, éthoxy, méthylthio, trifluorométhyle, trifluorométhoxy ou trifluorométhylthio, et on considère alors dans chaque cas comme hétérocycles :

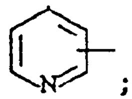 ; 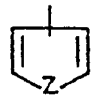 ; 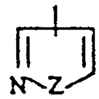 ; 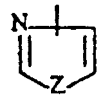 ;

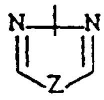 ; 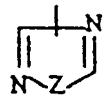 ; 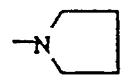 ; 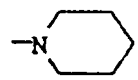 ;

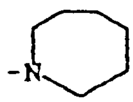 ; 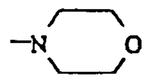 ou 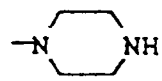 ,

Z désignant dans chaque cas l'oxygène ou le soufre,

$R^5$ représente en outre un groupe benzyle, phényléthyle, phénylpropyle, phénylbutyle, phénylpentyle, phénylhexyle, phénylheptyle, phénylcyanométhyle, phénylcyanéthyle, phénylcyanopropyle, benzoyle, phényle ou naphtyle, éventuellement à chaîne droite ou ramifiée, portant chacun le cas échéant dans la partie phényle 1 à 3 substituants, identiques ou différents, fluoro, chloro, bromo, hydroxy, cyano, nitro, méthyle, éthyle, n-propyle ou isopropyle, n-, iso-, sec.- ou tertiobutyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy,

caractérisé en ce qu'on fait réagir des chlorhydrates de chloro-formamidine de formule générale (II)

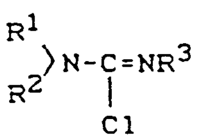 x HCl (II)

dans laquelle

$R^1$, $R^2$ et $R^3$ ont les définitions indiquées ci-dessus,

avec des hydrazides d'amide d'acide oxalique de formule générale (III)

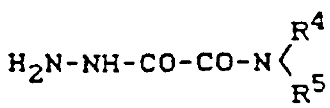 (III)

dans laquelle

$R^4$ et $R^5$ ont les définitions indiquées ci-dessus,

en présence d'un accepteur d'acide et en présence d'un diluant.

2. Procédé suivant la revendication 1, caractérisé en ce qu'on conduit la réaction à des températures de 0°C à 150°C.

3. Procédé suivant la revendication 1, caractérisé en ce qu'on utilise par mole de chlorhydrate de chloroformamidine de formule (II) entre 0,5 et 1,5 mole d'hydrazide d'amide d'acide oxalique de formule (III).

4. Procédé suivant la revendication 1, caractérisé en ce qu'on utilise par mole de chlorhydrate de chloroformamidine de formule (II) entre 0,5 et 1,5 mole d'hydrazide d'amide d'acide oxalique de formule (III) et 1 à 5 équivalents molaires d'un accepteur d'acide.